(19) Europäisches Patentamt
European Patent Office
Office européen des brevets

(11) Veröffentlichungsnummer: **0 006 506**
**B1**

# (12) EUROPÄISCHE PATENTSCHRIFT

(45) Veröffentlichungstag der Patentschrift: **16.09.81**

(51) Int. Cl.³: **C 07 D 401/12,** A 61 K 31/495

(21) Anmeldenummer: **79101827.8**

(22) Anmeldetag: **08.06.79**

(54) **1,2-Dihydro-chinolin-2-on-Derivate, Verfahren zu deren Herstellung sowie diese Verbindungen enthaltende Arzneimittel.**

(30) Priorität: **23.06.78 DE 2827566**

(43) Veröffentlichungstag der Anmeldung:
**09.01.80 Patentblatt 80/1**

(45) Bekanntmachung des Hinweises auf die Patenterteilung:
**16.09.81 Patentblatt 81/37**

(84) Benannte Vertragsstaaten:
**AT BE CH DE FR GB IT LU NL SE**

(56) Entgegenhaltungen:
**DE-A-1 932 384**
**DE-A-2 337 461**
**DE-A-2 631 080**
**GB-A-1 424 571**

(73) Patentinhaber: **BOEHRINGER MANNHEIM GMBH,**
**Sandhofer Strasse 112-132 Postfach 31 01 20,**
**D-6800 Mannheim 31-Waldhof (DE)**

(72) Erfinder: **Winter, Werner, Dr., Mannheimerstrasse 9,**
**D-6148 Heppenheim (DE)**
Erfinder: **Friebe, Walter-Gunar, Dr., Oberstrasse 13,**
**D-6100 Darmstadt (DE)**
Erfinder: **Kampe, Wolfgang, Dr., Zedernstrasse 49,**
**D-6805 Heddesheim (DE)**
Erfinder: **Roesch, Androniki, Dr., Am Oberen**
**Luisenpark 22, D-6800 Mannheim (DE)**
Erfinder: **Wilhelms, Otto-Henning, Dr.,**
**Odenwalstrasse 25/2, D-6941 Weinheim-Rittenweier (DE)**

**EP 0 006 506 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 1,2-Dihydro-chinolin-2-on-Derivate, Verfahren zu deren Herstellung sowie diese Verbindungen enthaltende Arzneimittel

Die vorliegende Erfindung betrifft Derivate des Dihydro-chinolin-2-ons der allgemeinen Formel I

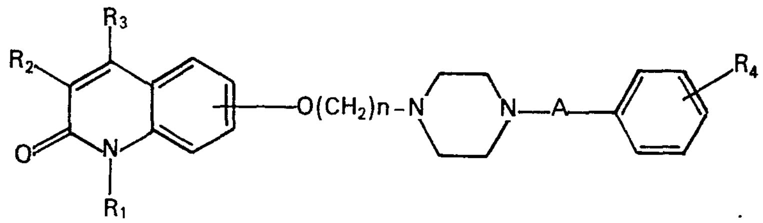

(I),

in der

$R_1$, $R_2$, $R_3$ die gleich oder verschieden sein können, Wasserstoff oder einen niederen Alkylrest,

$R_4$ Wasserstoff, Halogen, einen niederen Alkyl- oder einen niederen Alkoxy-Rest,

15n eine niedere Zahl von 2 bis 5 und

A einen Valenzstrich oder eine gegebenenfalls durch einen Phenylrest substituierte Methylengruppe bedeuten,

und deren pharmakologisch verträgliche Salze, Verfahren zu deren Herstellung, sowie ihre Verwendung bei der Bekämpfung von allergischen Krankheiten.

Weiterhin betrifft die Erfindung pharmazeutische Präparate mit einem Gehalt an Verbindungen der allgemeinen Formel I, sowie die Verwendung von Verbindungen der Formel I zur Herstellung solcher Präparate, wobei die erfindungsgemässen Substanzen gegebenenfalls in der Form von Salzen nichttoxischer anorganischer oder organischer Säuren Anwendung finden.

Die «niederen Alkyl-» sowie die «niederen Alkoxy-Reste» in der Definition der Substituenten $R_1$, $R_2$, $R_3$ und $R_4$ können geradkettig oder verzweigt sein und enthalten 1-6, vorzugsweise 1-4 Kohlenstoffatome.

Unter Halogen ist Fluor,Chlor und Brom zu verstehen, vorzugsweise Chlor.

Die neuen Verbindungen sowie ihre pharmakologisch verträglichen Salze weisen bei parenteraler und auch peroraler Gabe eine ausgeprägte, im Vergleich zum nächstliegenden Stand der Technik bessere antiallergische Wirkung auf und können daher besonders vorteilhaft bei der Bekämpfung von allergischen Krankheiten, beispielsweise von allergischem Asthma, Heuschnupfen und Urticaria, verwendet werden.

Zum Beweis hierfür wurden im pharmakologischen Test die passive cutane anaphylaktische Reaktion (PCA-Werte) in vivo bei Ratten für das Handelsprodukt HETRAZAN (Diethylcarbamazin; 1-Diethylcarbamoyl-4-methylpiperazin) und für die Verbindung des Beispiels 1 (4-Methyl-7-‹3-[4-(4-chlorbenzyl)-piperazin-1-yl]-propoxy›-2-oxo-1,2-dihydro-chinolin) ermittelt. Vergleichbare PCA-Werte werden mit der erfindungsgemässen Verbindung bei siebzigfach geringerer Dosis als mit dem Handelsprodukt erreicht. Anhand einer geeigneten Auswahl aus den beanspruchten Substanzen konnte aufgezeigt werden, dass auch die anderen erfindungsgemässen Verbindungen eine ebenso gute Inhibierung der passiven cutanen anaphylaktischen Reaktion bewirken. Die Hemmpotenz der beanspruchten Substanzklasse kann auch in vitro anhand der Antigen-induzierten Mastzellendegranulation überzeugend dargestellt werden.

Die neuen Verbindungen der allgemeinen Formel I lassen sich auf verschiedene Weise zu Substanzen weiterverarbeiten, die ebenfalls pharmakologische Wirksamkeit, insbesondere antiallergische oder antihypertensive Wirksamkeit, aufweisen. Sie stellen daher auch wertvolle Zwischenprodukte zur Herstellung von pharmakologisch wirksamen Stoffen dar.

Die erfindungsgemässen 1,2-Dihydro-chinolin-2-on-Derivate lassen sich nach an sich bekannten Methoden herstellen. Das bevorzugte Verfahren zur Herstellung der Verbindungen der allgemeinen Formel I

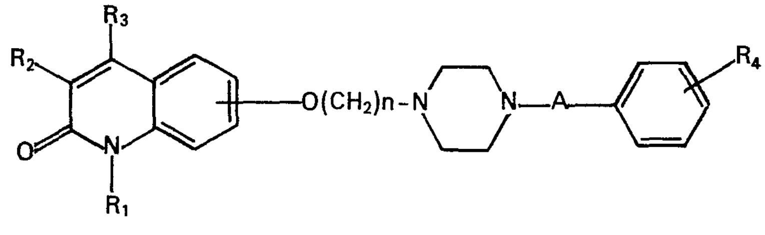

(I),

in der

$R_1$, $R_2$, $R_3$ die gleich oder verschieden sein können, Wasserstoff oder einen niederen Alkylrest,

$R_4$ Wasserstoff, Halogen, einen niederen Alkyl- oder einen niederen Alkoxy-Rest,

n eine niedere Zahl von 2 bis 5 und

A einen Valenzstrich oder eine gegebenenfalls durch einen Phenylrest substituierte Methylengruppe bedeuten,

sowie deren pharmakologisch verträglichen Salze ist dadurch gekennzeichnet, dass man in an sich bekannter Weise

a) ein 1,2-Dihydro-chinolin-2-on der allgemeinen Formel II

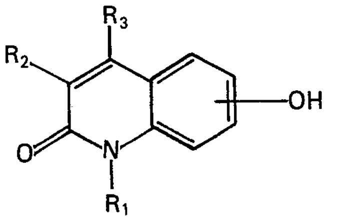

(II),

in der $R_1$, $R_2$ und $R_3$ die oben angegebene Bedeutung haben,
mit einem Piperazin-Derivat der allgemeinen Formel III

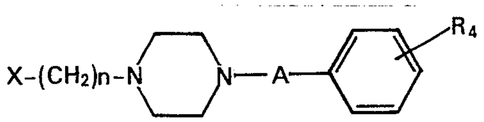

(III),

in der n, A und $R_4$ die oben angegebene Bedeutung haben und X einen reaktiven Rest darstellt, oder

b) ein 1,2-Dihydro-chinolin-2-on der allgemeinen Formel IV

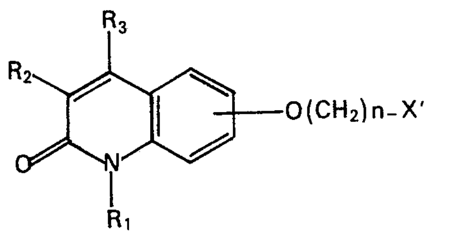

(IV),

in der $R_1$, $R_2$, $R_3$ und n die oben angegebene Bedeutung haben und X' einen reaktiven Rest darstellt,
mit einem Piperazin-Derivat der allgemeinen Formel V

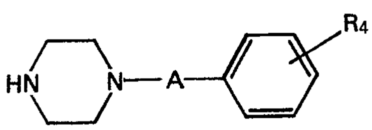

(V),

in der A und $R_4$ die oben angegebene Bedeutung haben,
reagieren lässt,
für den Fall, dass $R_1$ Wasserstoff bedeutet, gegebenenfalls nachträglich N-alkyliert und gewünschtenfalls die so erhaltenen Verbindungen der allgemeinen Formel I in pharmakologisch verträgliche Salze überführt.

Als reaktive Reste X beziehungsweise X' kommen alle üblichen Gruppen in Frage, die sich nucleophil verdrängen lassen. Besonders bevorzugt sind Chlor und Brom sowie die Mesyloxy- und Tosyloxy-Gruppe.

Die erfindungsgemässen Umsetzungen werden nach an sich bekannten Methoden durchgeführt. So lässt man beispielsweise bei Verfahrensvariante a) die Hydroxy-1,2-dihydro-chinolin-2-one der allgemeinen Formel II in Gegenwart eines Alkalialkoholats in einem geeigneten Lösungsmittel, wie Ethanol, Isopropanol oder vorzugsweise Ethoxy-ethanol, mit den Piperazinen der Formel III bei erhöhter Temperatur kondensieren. Als Lösungsmittel können auch Dimethylformamid, Dimethylsulfoxid oder Hexametapol eingesetzt werden.

Bei Verfahrensvariante b) wird die Umsetzung eines Dihydrochinolin-2-ons der Formel IV mit Piperazinen der Formel V in den genannten Lösungsmitteln vorteilhafterweise durch Zugabe eines tertiären Amins wie Triethylamin, einer Huenig-Base oder eines stark basischen Ionenaustauschers bewirkt, kann aber auch beispielsweise mit Kalium-t-butylat in Dimethylsulfoxid durchgeführt werden.

Die Ausgangsverbindungen der allgemeinen Formeln II, III, IV und V sind literaturbekannte Substanzen oder können in Analogie zu literaturbekannten Verfahren hergestellt werden.

Die pharmakologisch verträglichen Salze erhält man in üblicher Weise, z.B. durch Neutralisation der Verbindungen der Formel I mit nichttoxischen anorganischen oder organischen Säuren wie z.B. Salzsäure, Schwefelsäure, Phosphorsäure, Bromwasserstoffsäure, Essigsäure, Milchsäure, Zitronensäure, Äpfelsäure, Salicylsäure, Malonsäure, Maleinsäure oder Bernsteinsäure.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragées ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsmvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Geschmacks- und Süsstoffe enthalten. Für die äusserliche Anwendung können die erfindungsgemässen Substanzen I auch in Form von Pudern und Salben verwendet werden, sie werden dazu z.B. mit pulverförmigen, physiologisch verträglichen Verdünnungsmitteln bzw. üblichen Salbengrundlagen vermischt.

Ausser den in den nachstehenden Beispielen genannten Substanzen sind im Sinne der vorliegenden Anmeldung die folgenden Verbindungen bevorzugt:

4-Methyl-6-‹2-[4-(4-chlorbenzyl)-piperazin-1-yl]-ethoxy›-2-oxo-1,2-dihydro-chinolin;
4-Methyl-8-‹3-[4-(4-chlorbenzyl)-piperazin-1-yl]-propoxy›-2-oxo-1,2-dihydrochinolin; 65,7% Ausbeute, Schmp. 144°C
1,4-Dimethyl-8-‹3-[4-(4-chlorbenzyl)-piperazin-1-yl]-propoxy›-2-oxo-1,2-dihydro-chinolin.

Daraus und aus den nachstehenden Beispielen ergibt sich, dass sich der Ethersubstituent am Chinolinring in Formel I vorzugsweise in der 6-, 7- oder 8-Stellung, insbesondere in der 6- und 7-Stellung, befindet und dass n vorzugsweise die Zahlen 2 und 3 bedeutet, wobei die Zahl 3 besonders bevorzugt ist.

Die nachstehenden Beispiele sollen die Erfindung näher erläutern, ohne sie jedoch zu beschränken.

Die Struktur der folgenden Beispiele ist durch CHN-Analyse, IR-, UV-, NMR- und Massenspektrum gesichert.

Beispiel 1
4-Methyl-7-‹3-[4-(4-chlorbenzyl)-piperazin-1-yl]-propoxy›-2-oxo-1,2-dihydro-chinolin

2,3 g Natrium (0,1 mol) werden in 320 ml Ethoxy-ethanol gelöst und anschliessend mit 17,5 g 4-Methyl-7-hydroxy-2-oxo-1,2-dihydro-chinolin (0,1 mol) versetzt. Man rührt 15 min nach, tropft dann 31,6 g 3-[4-(4-Chlorbenzyl)-piperazinyl-1-]-propylchlorid (0,11 mol) in 30 ml Ethoxy-ethanol zu und erwärmt 10 h auf 90°C. Das Lösungsmittel wird danach im Vacuum weitgehend entfernt. Den Rückstand versetzt man mit Wasser, extrahiert mit Methylenchlorid und dampft die organische Phase nach dem Trocknen ein. Der Eindampfrückstand wird durch Ausrühren mit Ether gereinigt. Es verbleiben 22,9 g (53,8% d.Th.) 4-Methyl-7-‹3-[4-(4-chlorbenzyl)-piperazin-1-yl]-propoxy›-2-oxo-1,2-dihydro-chinolin vom Schmelzpunkt 170–171°C.

Man löst die Base in Methylenchlorid und etwas Methanol und fällt durch Zugabe von etherischer Salzsäure und anschliessender Verdünnung mit Ether das entsprechende Dihydrochlorid vom Schmelzpunkt 271–272°C.

Beispiel 2
3,4-Dimethyl-7-‹3-[4-(4-chlorbenzyl)-piperazin-1-yl]-propoxy›-2-oxo-1,2-dihydro-chinolin

Aus 9,46 g (0,05 mol) 3,4-Dimethyl-7-hydroxy-2-oxo-1,2-dihydro-chinolin und 15,8 g (0,055 mol) 3-[4-(4-Chlorbenzyl)-piperazinyl-1]-propylchlorid erhält man nach der in Beispiel 1 beschriebenen Verfahrensvariante 12,8 g (58,2% d.Th.) der gewünschten Titelverbindung vom Schmelzpunkt 143–144°C.

Das entsprechende Hydrochlorid schmilzt bei 273–274°C (Wassergehalt 4,2%).

Beispiel 3
1,4-Dimethyl-7-‹3-[4-(4-chlorbenzyl)-piperazin-1-yl]-propoxy›-2-oxo-1,2-dihydro-chinolin

Aus 9,46 g 1,4-Dimethyl-7-hydroxy-2-oxo-1,2-dihydro-chinolin erhält man mit 15,8 3-[4-(4-Chlorbenzyl)-piperazin-1-yl]-propylchlorid nach der Arbeitsvorschrift des Beispiels 1 15,2 g (63,8% d.Th.) der gewünschten Titelverbindung als Mono-Hydrochlorid. Die Thermoanalyse zeigt einen Schmelzpunkt von 189°C. Ein entsprechendes Dihydrochlorid schmilzt bei 260–263°C.

Beispiel 4
4-Methyl-7-‹3-[4-(3-chlorphenyl)-piperazin-1-yl]-propoxy›-2-oxo-1,2-dihydro-chinolin

In Analogie zu Beispiel 1 erhält man aus 8,76 g 4-Methyl-7-hydroxy-2-oxo-1,2-dihydro-chinolin (0,05 mol) und 15 g 3-[4-(3-Chlorphenyl)-piperazin-1-yl]-propylchlorid (0,055 mol) mit 1,55 g Natrium (0,05 mol) in 160 ml Ethoxy-ethanol nach fünfstündiger Reaktion bei 90°C die Titelverbindung vom Schmelzpunkt 212–214°C. Ausbeute: 10,5 g (51% d.Th.)

Beispiel 5
4-Methyl-6-‹3-[4-(4-chlorbenzyl)-piperazin-1-yl]-propoxy›-2-oxo-1,2-dihydro-chinolin

Man löst 0,69 g (0,03 mol) Natrium in 75 ml Isopropanol, versetzt mit 4,89 g (0,03 mol) 4-Methyl-6-hydroxy-2-oxo-1,2-dihydro-chinolin und rührt 10 min bei Raumtemperatur. Nach Zugabe von 9,5 g (0,033 mol) 3-[4-(4-Chlorbenzyl)-piperazin-1-yl]-propylchlorid in 25 ml Isopropanol wird 16 h zum Sieden erhitzt; danach engt man im Vacuum ein, nimmt den Rückstand in Methylenchlorid auf und schüttelt mit 1 N Natronlauge aus. Die getrocknete organische Phase engt man danach ein, verreibt den Rückstand mit Ether und kristallisiert das Festprodukt aus Alkohol um. Es werden 6,25 g (49% d.Th.) der Titelverbindung vom Schmelzpunkt 195–197°C isoliert.

Beispiel 6
4-Methyl-6-‹3-[-(4-fluorbenzyl)-piperazin-1-yl]-propoxy›-2-oxo-1,2-dihydro-chinolin

In Analogie zum Beispiel 5 erhält man durch entsprechende Umsetzung von 4-Methyl-6-hydroxy-2-oxo-1,2-dihydro-chinolin mit 3-[4-Fluorbenzyl)-piperazin-1-yl]-propylchlorid die Titelverbindung als Base vom Schmelzpunkt 178–180°C (aus Alkohol).

Beispiel 7
4-Methyl-6-‹3-[4-(4-tert.butyl-benzyl)-piperazin-1-yl]-propoxy›-2-oxo-1,2-dihydro-chinolin

In Analogie zu Beispiel 5 erhält man durch Umsetzung von 4-Methyl-6-hydroxy-2-oxo-1,2-dihydro-chinolin mit 3-[4-(4-tert.Butyl-benzyl)-piperazin-1-yl]-propylchlorid die Titelverbindung als Base vom Schmelzpunkt 171–172°C (aus Essigester).

Beispiel 8
4-Methyl-6-‹3-[4-(4-methylbenzyl)-piperazin-1-yl]-propoxy›-2-oxo-1,2-dihydro-chinolin

In Analogie zu Beispiel 5 wird 4-Methyl-6-hydroxy-2-oxo-1,2-dihydro-chinolin mit 3-[4-(4-Methylbenzyl)-piperazin-1-yl]-propylchlorid umgesetzt. Man erhält die Titelverbindung als Base vom Schmelzpunkt 162–163°C (aus Ether).

Beispiel 9
6-‹3-[4-(4-Chlorbenzyl)-piperazin-1-yl]-propoxy›-2-oxo-1,2-dihydro-chinolin

4,8 g (0,03 mol) 6-Hydroxy-2-oxo-1,2-dihydrochinolin werden in 60 ml Wasser und 30 ml 1 N Natronlauge gelöst. Anschliessend engt man die Lösung weitgehend ein, nimmt den Rückstand in 75 ml Dimethylformamid auf und versetzt mit 14,3 g (0,05 mol) 3-[4-(4-Chlorbenzyl)-piperazin-1-yl]-propylchlorid. Nach vierstündigem Rühren bei 100 °C wird das Lösungsmittel im Vacuum entfernt und der Rückstand in Methylenchlorid aufgenommen. Man schüttelt mit 1 N Natronlauge und Wasser aus, trocknet die organische Phase und engt im Vacuum ein. Der Rückstand wird anschliessend mit Essigester verrieben und aus Alkohol umkristallisiert.

Man isoliert die Titelverbindung in einer Ausbeute von 6,3 g (51% d.Th.). Die Base hat einen Schmelzpunkt von 180–182 °C.

Beispiel 10
4-Methyl-6-‹3-[4-(2-propoxy-benzyl)-piperazin-1-yl]-propoxy›-2-oxo-1,2-dihydro-chinolin

In Analogie zu Beispiel 9 setzt man 4-Methyl-6-hydroxy-2-oxo-1,2-dihydro-chinolin mit 3-[4-(2-Propoxy-benzyl)-piperazin-1-yl]-propylchlorid um. In 53%iger Ausbeute erhält man die Titelverbindung als ölige Base. Aus acetonischer Lösung wird durch Zugabe von etherischer Salzsäure ein entsprechendes Hydrochlorid hergestellt, das in amorpher Form mit einem Schmelzpunkt von ca. 140–145 °C anfällt.

Beispiel 11
1,4-Dimethyl-6-‹3-[4-(4-chlorbenzyl)-piperazin-1-yl]-propoxy›-2-oxo-1,2-dihydro-chinolin

In Analogie zu Beispiel 3 wird 1,4-Dimethyl-6-hydroxy-2-oxo-1,2-dihydro-chinolin mit 3-[4-(4-Chlorbenzyl)-piperazin-1-yl]-propylchlorid umgesetzt. Man erhält die Titelverbindung als Hydrochlorid vom Schmelzpunkt 215–220 °C (Zers.).

Beispiel 12
4-Methyl-7-‹3-[4-(3-methoxybenzyl)-piperazin-1-yl]-propoxy›-2-oxo-1,2-dihydro-chinolin

In Analogie zu Beispiel 1 setzt man 8,76 g (0,05 mol) 4-Methyl-7-hydroxy-2-oxo-1,2-dihydro-chinolin mit 15,5 g (0,055 mol) 3-[4-(3-Methoxybenzyl)piperazin-1-yl]propylchlorid in Ethoxyethanol durch vierstündiges Erwärmen auf 90 °C um. Nach der Aufarbeitung wird das Rohprodukt mit Ether verrieben. Es verbleiben 12,2 g (57,9% d.Th.) der gewünschten Verbindung vom Schmelzpunkt 149–150 °C. Ein entsprechendes Hydrochlorid schmilzt bei 265–268 °C.

Beispiel 13
4-Methyl-7-‹3-[4-(4-methoxybenzyl)-piperazin-1-yl]-propoxy›-2-oxo-1,2-dihydro-chinolin

Durch analoge Umsetzung wie im Beispiel 12 erhält man unter Verwendung von 3-[4-(4-Methoxy-benzyl)piperazin-1-yl]propylchlorid die gewünschte Verbindung als Base (Ausbeute: 11 g = 52% d.Th.) vom Schmelzpunkt 167–168 °C. Das entsprechende Dihydrochlorid schmilzt bei 248–250 °C.

Beispiel 14
4-Methyl-7-‹3-[4-(2-methoxybenzyl)-piperazin-1-yl]-propoxy›-2-oxo-1,2-dihydro-chinolin

In Analogie zu Beispiel 12 werden 8,76 g (0,05 mol) 4-Methyl-7-hydroxy-2-oxo-1,2-dihydro-chinolin mit 15,5 g (0,055 mol) 3-[4-(2-Methoxybenzyl)-piperazin-1-yl]-propylchlorid umgesetzt (4 h bei 90 °C). Man erhält die Titelverbindung als Base in einer Ausbeute von 11,7 g (55,5% d.Th.) vom Schmelzpunkt 151–152 °C. Das entsprechende Hydrochlorid schmilzt bei 265–266 °C.

Beispiel 15
4-Methyl-7-[3-(4-benzyl-piperazinyl-2)-propoxy]-2-oxo-1,2-dihydro-chinolin

In Analgoie zu Beispiel 12 setzt man 8,76 g 4-Methyl-7-hydroxy-2-oxo-1,2-dihydro-chinolin mit 3-(4-Benzyl-piperazin-1-yl)-propylchlorid innerhalb 4 h bei 90 °C um. Die Titelverbindung wird als Base in einer Ausbeute von 11,4 g (58,3% d.Th.) mit einem Schmelzpunkt von 185–186 °C isoliert. Das entsprechende Hydrochlorid schmilzt bei 279–281 °C.

Beispiel 16
3-n-Butyl-4-methyl-7-‹3-[4-(4-chlorbenzyl)-piperazin-1-yl]-propoxy›-2-oxo-1,2-dihydro-chinolin

Gemäss Beispiel 1 setzt man 3-n-Butyl-4-methyl-7-hydroxy-2-oxo-1,2-dihydro-chinolin mit 3-[4-(4-Chlorbenzyl)-piperazin-1-yl]-propylchlorid in Ethoxy-ethanol um. Die Titelverbindung wird als Base in 57,5%iger Ausbeute isoliert. Schmelzpunkt 148–149 °C.

Zur gleichen Verbindung gelangt man, wenn 7-(3-Chlorpropoxy)-3-n-butyl-4-methyl-2-oxo-1,2-dihydro-chinolin (Fp. 203 °C) mit 4-(4-Chlorbenzyl)-piperazin in Tetrahydrofuran in Gegenwart einer tertiären Base, wie beispielsweise N-Ethyl-di-isopropylamin, 8 h zum Sieden erhitzt wird.

Beispiel 17
4-Methyl-7-‹3-[4-(diphenylmethyl)-piperazin-1-yl]-propoxy›-2-oxo-1,2-dihydro-chinolin

In Analogie zu Beispiel 1 setzt man 4-Methyl-7-hydroxy-2-oxo-1,2-dihydro-chinolin (8,76 g) mit 3-[4-(Diphenylmethyl)-piperazin-1-yl)-propylchlorid (18,1 g) in Ethoxy-ethanol um.

Man erhält die Titelverbindung als Base (15 g) mit einem Schmelzpunkt von 208–210 °C. Die Ausbeute beträgt 64% d.Th.

**Patentansprüche** für die Vertragsstaaten BE, CH, DE, FR, GB, IT, LU, NL, SE

1. 1,2-Dihydro-chinolin-2-one der allgemeinen Formel I

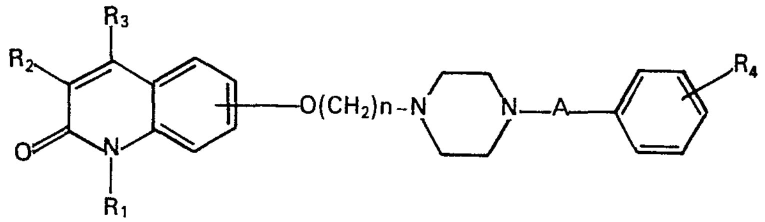

(I),

in der
$R_1$,$R_2$,$R_3$ die gleich oder verschieden sein können, Wasserstoff oder einen Alkylrest mit 1-6 Kohlenstoffatomen,
$R_4$ Wasserstoff, Halogen, einen Alkyl- oder einen Alkoxy-Rest mit jeweils 1-6 Kohlenstoffatomen,
n eine Zahl von 2 bis 5 und
A einen Valenzstrich oder eine gegebenenfalls durch einen Phenylrest substituierte Methylengruppe bedeuten,
und deren pharmakologisch verträglichen Salze.

2. Verbindungen gemäss Anspruch 1, in denen $R_1$, $R_2$, $R_3$, $R_4$ und A die angegebene Bedeutung haben, n die Zahlen 2 oder 3 darstellen und die Position des Ethersubstituenten in 6-, 7- oder 8-Stellung des Chinolin-Ringes ist.

3. Verbindungen gemäss Anspruch 1 oder 2, in denen $R_1$, $R_2$, $R_3$, $R_4$ und A die angegebene Bedeutung haben, n die Zahl 3 darstellt und die Position des Ethersubstituenten in 6- oder 7-Stellung des Chinolin-Ringes ist.

4. Verfahren zur Herstellung von 1,2-Dihydrochinolin-2-onen gemäss den Ansprüchen 1 bis 3 mit den dort angegebenen Bedeutungen der Substituenten und deren pharmakologisch verträglichen Salzen, dadurch gekennzeichnet, dass man in an sich bekannter Weise
a) ein 1,2-Dihydro-chinolin-2-on der allgemeinen Formel II

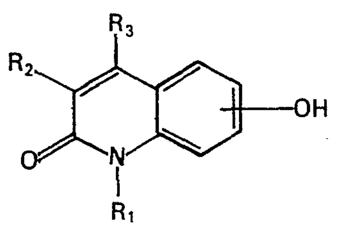

(II),

in der $R_1$, $R_2$ und $R_3$ die oben angegebene Bedeutung haben,
mit einem Piperazin-Derivat der allgemeinen Formel III

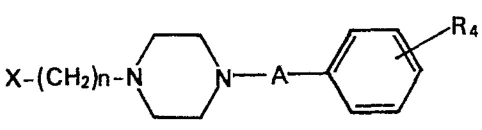

(III),

in der n, A und $R_4$ die oben angegebene Bedeutung haben und X einen reaktiven Rest darstellt, oder
b) ein 1,2-Dihydro-chinolin-2-on der allgemeinen Formel IV

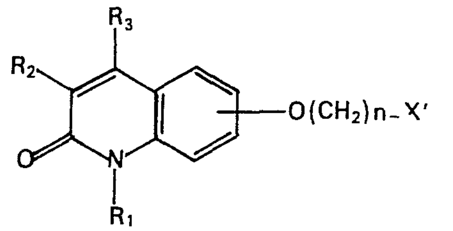

(IV),

in der $R_1$, $R_2$, $R_3$ und n die oben angegebene Bedeutung haben und X′ einen reaktiven Rest darstellt,

mit einem Piperazin-Derivat der allgemeinen Formel V

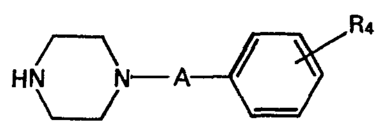

(V),

in der A und $R_4$ die oben angegebene Bedeutung haben, reagieren lässt,
für den Fall, dass $R_1$ Wasserstoff bedeutet, gegebenenfalls nachträglich N-alkyliert und gewünschtenfalls die so erhaltenen Verbindungen der allgemeinen Formel I in pharmakologisch verträgliche Salze überführt.

5. Verbindungen gemäss den Ansprüchen 1 bis 3 zur Verwendung bei der Bekämpfung allergischer Krankheiten.

6. Arzneimittel, gekennzeichnet durch einen Gehalt an Verbindungen gemäss den Ansprüchen 1 bis 3 und üblichen Träger- und Hilfsstoffen.

**Patentansprüche** für den Vertragsstaat AT

1. Verfahren zur Herstellung von 1,2-Dihydrochinolin-2-onen der allgemeinen Formel I

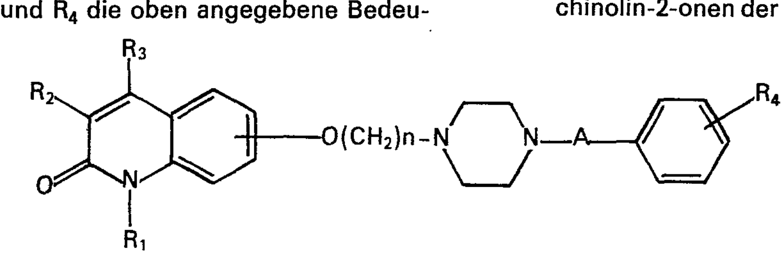

(I),

in der
$R_1$, $R_2$, $R_3$ die gleich oder verschieden sein können, Wasserstoff oder einen Alkylrest mit 1–6 Kohlenstoffatomen,
$R_4$ Wasserstoff, Halogen, einen niederen Alkyl- oder einen Alkoxy-Rest mit jeweils 1–6 Kohlenstoffatomen,
n eine Zahl von 2 bis 5 und
A einen Valenzstrich oder eine gegebenenfalls durch einen Phenylrest substituierte Methylengruppe bedeuten,
und deren pharmakologisch verträglichen Salze, dadurch gekennzeichnet, dass man in an sich bekannter Weise
a) ein 1,2-Dihydro-chinolin-2-on der allgemeinen Formel II

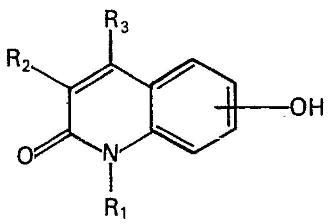

(II),

in der $R_1$, $R_2$ und $R_3$ die oben angegebene Bedeutung haben,
mit einem Piperazin-Derivat der allgemeinen Formel III

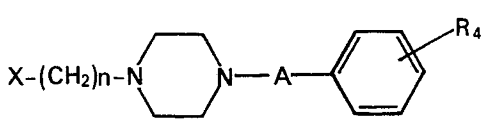

(III),

in der n, A und $R_4$ die oben angegebene Bedeutung haben und X einen reaktiven Rest darstellt,
oder
b) ein 1,2-Dihydro-chinolin-2-on der allgemeinen Formel IV

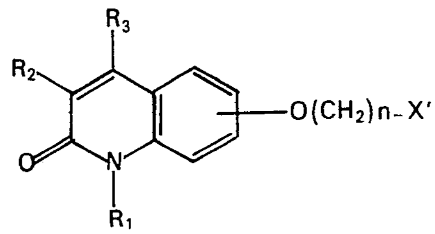

(IV),

in der $R_1$, $R_2$, $R_3$ und n die oben angegebene Bedeutung haben und X′ einen reaktiven Rest darstellt,
mit einem Piperazin-Derivat der allgemeinen Formel V

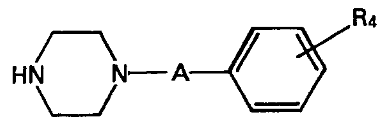

(V),

in der A und $R_4$ die oben angegebene Bedeutung haben,
reagieren lässt,
für den Fall, dass $R_1$ Wasserstoff bedeutet, gegebenenfalls nachträglich N-alkyliert und gewünschtenfalls die so erhaltenen Verbindungen der allgemeinen Formel I in pharmakologisch verträgliche Salze überführt.

2. Verfahren gemäss Anspruch 1 zur Herstellung von Verbindungen der Formel I, in denen $R_1$, $R_2$, $R_3$, $R_4$ und A die angegebene Bedeutung haben, n die Zahlen 2 oder 3 darstellen und die Position des Ethersubstituenten in 6-, 7- oder 8-Stellung des Chinolin-Ringes ist.

3. Verfahren gemäss Anspruch 1 zur Herstellung von Verbindungen der Formel I, in denen $R_1$, $R_2$, $R_3$, $R_4$ und A die angegebene Bedeutung haben, n die Zahl 3 darstellt und die Position des Ethersubstituenten in 6- oder 7-Stellung des Chinolin-Ringes ist.

**Revendications** pour les Etats contractants BE, CH, DE, FR, GB, IT, LU, NL, SE

1. Dérivés de 1,2-dihydroquinoléine-2-one de formule générale I

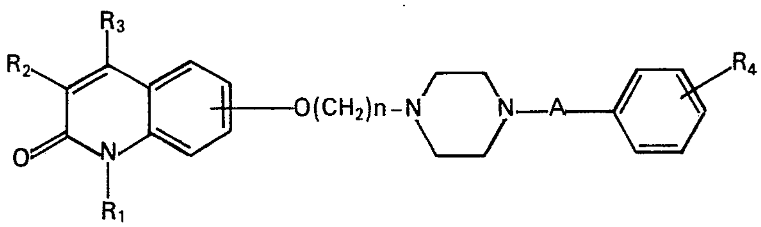

(I),

dans laquelle:
$R_1$, $R_2$ et $R_3$, pouvant être identiques ou différents, sont de l'hydrogène ou un reste alkyle ayant de 1 à 6 atomes de carbone,
$R_4$ est de l'hydrogène ou un halogène, un reste alkyle ou alcoxyle ayant de 1 à 6 atomes de carbone
n est un nombre compris entre 2 et 5, et,
A est un trait de valence ou un groupe méthylène éventuellement substitué par un groupe phényle;
ainsi que leurs sels pharmacologiquement tolérables.

2. Dérivés selon la revendication 1, dans lesquels $R_1$, $R_2$, $R_3$, $R_4$ et A ont la signification indiquée ci-dessus, n est le nombre 2 ou 3, et le substituant éther occupe la position 6, 7 ou 8 sur le

noyau quinoléinique.

3. Dérivés selon la revendication 1 ou 2, dans lesquels $R_1$, $R_2$, $R_3$, $R_4$ et A ont la signification ci-dessus indiquée, n est le nombre 3 et le substituant éther occupe la position 6 ou 7 du noyau quinoléinique.

4. Procédé pour la préparation de dérivés de la 1,2-dihydroquinoléine-2-one selon les revendications 1 à 3 dans lesquels les substituants ont la signification ci-dessus indiquée ainsi que leurs sels pharmacologiquement tolérables, caractérisé en ce que l'on fait réagir de façon en soi connue,

a) un dérivé de la 1,2-dihydroquinoléine-2-one de formule générale II

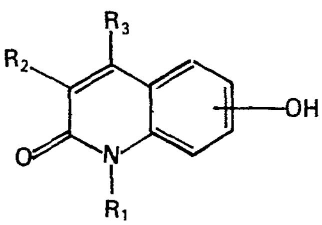

(II),

dans laquelle $R_1$, $R_2$ et $R_3$ ont la signification ci-dessus indiquée, avec un dérivé de pipérazine de formule générale III

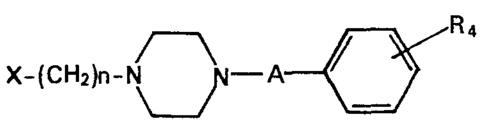

(III),

dans laquelle n, A et $R_4$ ont la signification donnée ci-dessus et X est un reste réactif, ou

b) un dérivé de la 1,2-dihydroquinoléine-2-one de formule générale IV

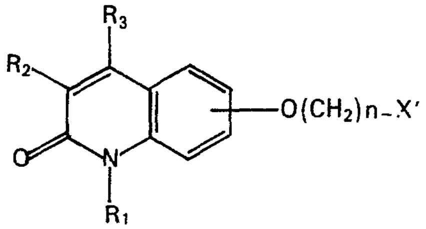

(IV),

dans laquelle $R_1$, $R_2$, $R_3$ et n ont la signification ci-dessus indiquée et X' est un reste réactif, avec un dérivé de pipérazine de formule générale V

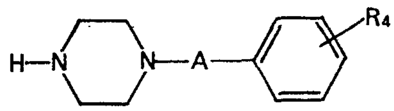

(V)

dans laquelle A et $R_4$ ont la signification indiquée ci-dessus, et en ce que dans le cas où $R_1$ est de l'hydrogène, on effectue éventuellement ultérieurement une N-alkylation, et si l'on désire on transforme les composés de formule I ainsi obtenus en sels pharmacologiquement tolérables.

5. Utilisation des dérivés selon les revendications 1 à 3 pour la lutte contre les maladies allergiques.

6. Médicaments caractérisés en ce qu'ils contiennent des composés selon les revendications 1 à 3 ainsi que des substances auxiliaires et de support.

**Revendications** pour l'Etat contractant AT

1. Procédé pour la préparation de dérivés de la 1,2-dihydro-quinoléine-2-one de formule générale I

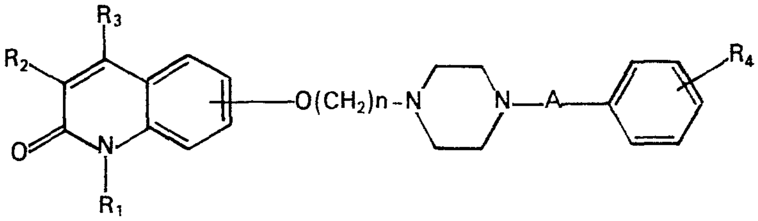

(I),

dans laquelle:

$R_1$, $R_2$ et $R_3$, pouvant être identiques ou différents, sont de l'hydrogène ou un reste alkyle ayant de 1 à 6 atomes de carbone,

$R_4$ est de l'hydrogène ou un halogène, un reste alkyle ou alcoxyle ayant de 1 à 6 atomes de carbone,

n est un nombre compris entre 2 et 5, et

A est un trait de valence ou un groupe méthylène éventuellement substitué par un groupe phényle ainsi que leurs sels pharmacologiquement tolérables, caractérisé en ce que l'on fait réagir de façon en soi connue,

a) un dérivé de la 1,2-dihydroquinoléine-2-one de formule générale II

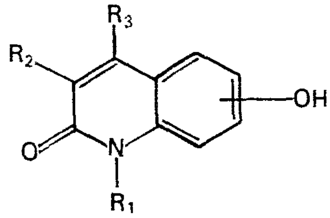

(II),

dans laquelle $R_1$, $R_2$ et $R_3$ ont la signification ci-dessus indiquée, avec un dérivé de pipérazine de formule générale III

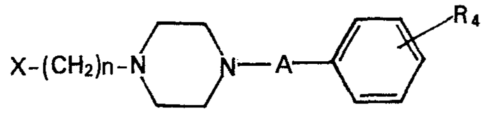

(III),

dans laquelle n, A et $R_4$ ont la signification donnée ci-dessus et X est un reste réactif, ou
b) un dérivé de la 1,2-dihydroquinoléine-2-one de formule générale IV

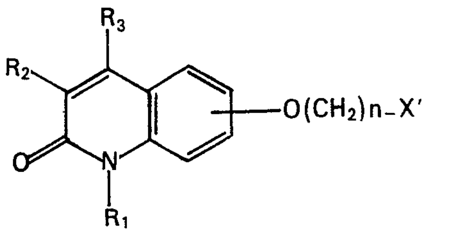

(IV),

dans laquelle $R_1$, $R_2$, $R_3$ et n ont la signification ci-dessus indiquée et X′ est un reste réactif, avec un dérivé de pipérazine de formule générale V

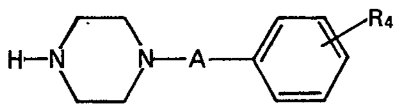

(V)

dans laquelle A et $R_4$ ont la signification indiquée ci-dessus, et en ce que dans le cas où $R_1$est de l'hydrogène, on effectue éventuellement ultérieurement une N-alkylation, et si l'on désire on transforme les composés de formule I ainsi obtenus en sels pharmacologiquement tolérables.

2. Procédé selon la revendication 1 pour la préparation de dérivés de formule I, dans lesquels $R_1$, $R_2$, $R_3$, $R_4$ et A ont la signification indiquée ci-dessus, n est le nombre 2 ou 3, et le substituant éther occupe la position 6, 7 ou 8 sur le noyau quinoléinique.

3. Procédé selon la revendication 1 pour la préparation de dérivés de formule I, dans lesquels $R_1$, $R_2$, $R_3$, $R_4$ et A ont la signification ci-dessus indiquée, n est le nombre 3 et le substituant éther occupe la position 6 ou 7 du noyau quinoléinique.

**Claims** for the Contracting states BE, CH, DE, FR, GB, IT, LU, NL, SE

1. 1,2-Dihydroquinolin-2-one derivatives of the general formula I

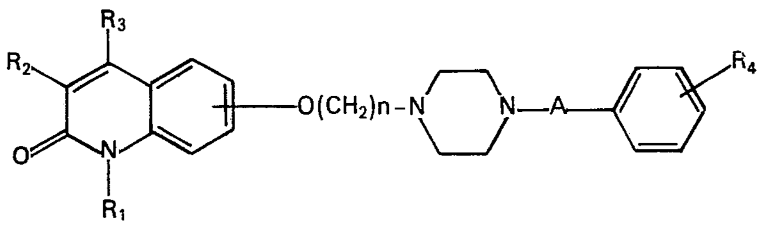

(I),

in which $R_1$, $R_2$ and $R_3$, which can be the same or different, signify hydrogen or an alkyl radical with 1-6 carbon atoms, $R_4$ hydrogen, halogen, an alkyl or an alkoxy radical, each with 1-6 carbon atoms, n a number from 2 to 5 and A a valency bond or a methylene radical optionally substituted by a phenyl radical; and their pharmacologically acceptable salts.

2. Compounds according to claim 1, in which $R_1$, $R_2$, $R_3$, $R_4$ and A have the given meaning, n represents the number 2 or 3 and the position of the ether substituent is in the 6-, 7- or 8-position of the quinoline ring.

3. Compounds according to claim 1 or 2, in which $R_1$, $R_2$, $R_3$, $R_4$ and A have the given meaning, n represents the number 3 and the position of the ether substituent is in the 6- or 7-position of the quinoline ring.

4. Process for the preparation of 1,2-dihydroquinolin-2-one derivatives of claims 1 to 3 with the there given meanings of the substituents and of their pharmacologically compatible salts, characterised in that, in per se known manner, one allows
a) a 1,2-dihydroquinolin-2-one of the general formula II

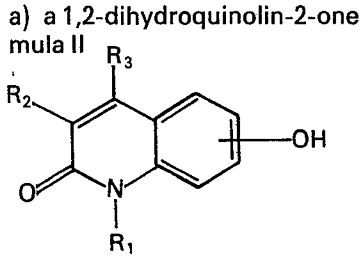

(II),

in which $R_1$, $R_2$ and $R_3$ have the above-given meaning, to react with a piperazine derivative of the general formula III

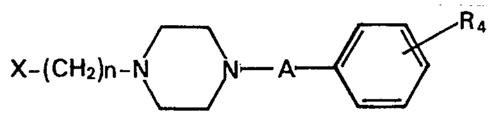

(III),

in which n, A and $R_4$ have the above-given meaning and X represents a reactive residue, or
b) a 1,2-dihydroquinolin-2-one of the general formula IV

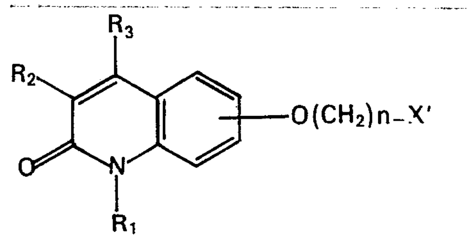

(IV),

in which $R_1$, $R_2$, $R_3$ and n have the above-given meaning and X′ represents a reactive residue, to react with a piperazine derivative of the general formula V

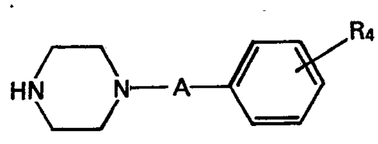

(V),

in which A and $R_4$ have the above-given meaning, for the case in which $R_1$ signifies hydrogen, possibly subsequently N-alkylates and, if desired, converts the so obtained compounds of the general formula I into pharmacologically acceptable salts.

5. Compounds according to claims 1 to 3 for use in the combating of allergic diseases.

6. Medicaments, characterised by a content of compounds according to claims 1 to 3 and conventional carrier and adjuvant materials.

**Claims** for the Contracting state AT

1. Process for the preparation of 1,2-Dihydroquinolin-2-one derivatives of the general formula I

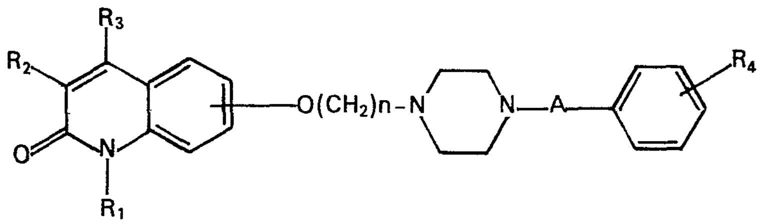

(I),

in which $R_1$, $R_2$ and $R_3$, which can be the same or different, signify hydrogen or an alkyl radical with 1-6 carbon atoms, $R_4$ hydrogen, halogen, an alkyl or an alkoxy radical, each with 1-6 carbon atoms, n a number from 2 to 5 and A a valency bond or a methylene radical, and of their pharmacologically compatible salts, characterised in that, in per se known manner, one allows

a) a 1,2-dihydroquinolin-2-one of the general formula II

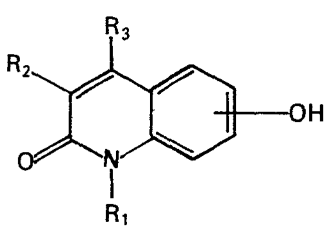

(II),

in which $R_1$, $R_2$ and $R_3$ have the above-given meaning, to react with a piperazine derivative of the general formula III

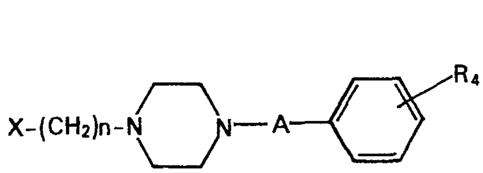

(III),

in which n, A and $R_4$ have the above-given meaning and X represents a reactive residue, or

b) a 1,2-dihydroquinolin-2-one of the general formula IV

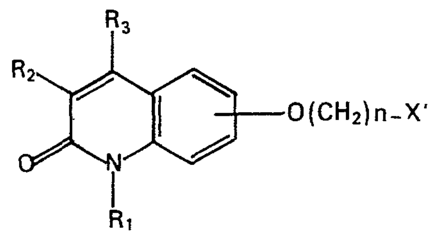

(IV),

in which $R_1$, $R_2$, $R_3$ and n have the above-given meaning and X′ represents a reactive residue, to react with a piperazine derivative of the general formula V

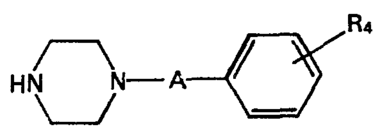

(V),

in which A and $R_4$ have the above-given meaning, for the case in which $R_1$ signifies hydrogen, possibly subsequently N-alkylates and, if desired, converts the so obtained compounds of the general formula I into pharmacologically acceptable salts.

2. Process according to claim 1 for the preparation of compounds of formula I, in which $R_1$, $R_2$, $R_3$, $R_4$ and A have the given meaning, n represents the number 2 or 3 and the position of the ether substituent is in the 6-, 7- or 8-position of the quinoline ring.

3. Process according to claim 1 for the preparation of compounds of formula I, in which $R_1$, $R_2$, $R_3$, $R_4$ and A have the given meaning, n represents the number 3 and the position of the ether substituent is in the 6- or 7-position of the quinoline ring.